(19) Europäisches Patentamt
European Patent Office
Office européen des brevets

(11) Publication number : **0 430 371 B1**

# (12) EUROPEAN PATENT SPECIFICATION

(45) Date of publication of patent specification : **17.11.94 Bulletin 94/46**

(51) Int. Cl.$^5$ : **G01N 33/543,** G01N 33/94, G01N 33/78

(21) Application number : **90203120.2**

(22) Date of filing : **26.11.90**

## (54) Dry immunoassay analytical element comprising monodispersed beads.

(30) Priority : **30.11.89 US 444079**

(43) Date of publication of application : **05.06.91 Bulletin 91/23**

(45) Publication of the grant of the patent : **17.11.94 Bulletin 94/46**

(84) Designated Contracting States : **AT BE CH DE ES FR GB GR IT LI LU NL SE**

(56) References cited : **EP-A- 0 013 156**

(73) Proprietor : **EASTMAN KODAK COMPANY**
**343 State Street**
**Rochester New York 14650-2201 (US)**

(72) Inventor : **Mauck, Linda Ann, c/o EASTMAN KODAK COMP.**
**Patent Department,**
**343 State Street**
**Rochester, New York 14650 (US)**
Inventor : **Danielson, Susan Jean, c/o EASTMAN KODAK COMP.**
**Patent Department,**
**343 State Street**
**Rochester, New York 14650 (US)**
Inventor : **Sundberg, Michael William, c/o EASTMAN KODAK COMP.**
**Patent Department,**
**343 State Street**
**Rochester, New York 14650 (US)**
Inventor : **Dappen, Glenn Marshall, c/o EASTMAN KODAK COMP.**
**Patent Department,**
**343 State Street**
**Rochester, New York 14650 (US)**
Inventor : **Eikenberry, Jon Nathan, c/o EASTMAN KODAK COMP.**
**Patent Department,**
**343 State Street**
**Rochester, New York 14650 (US)**

(74) Representative : **Nunney, Ronald Frederick Adolphe et al**
**Kodak Limited**
**Patent Department**
**Headstone Drive**
**Harrow Middlesex HA1 4TY (GB)**

EP 0 430 371 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to clinical chemistry; to an element for immunoassays and to methods for the determination of an immunologically reactive ligand.

Immunoassays, which take advantage of natural immunological reactions, have found widespread use as analytical techniques in clinical chemistry. Because of the specificity of the reactions, they are particularly advantageous in quantifying biological analytes that are present in very low concentration. Such analytes (called ligands herein) include, for example, antibodies, therapeutic drugs, narcotics, enzymes, hormones, proteins, etc.

In competitive binding assays, a labeled ligand analog (identified as ligand analog herein) is placed in competition with unlabeled ligand for reaction with a fixed amount of the appropriate binding material (called a receptor herein). Unknown concentrations of the ligand can be determined from the measured signal of either the bound or unbound (that is, free) ligand analog. The reaction proceeds as follows:

$$\text{ligand + ligand analog + receptor} \rightleftarrows \text{ligand-receptor + ligand analog-receptor.}$$

Conventional labels include radioactive tags, enzymes, chromophores, fluorophores, stable free radicals, and enzyme cofactors, inhibitors and allosteric effectors.

U.S. Patent 4,670,381 disclosed a dry immunoassay analytical element. In this element separation of bound and free ligand is accomplished by using a beaded spreading layer with a porosity such that spreading of the liquid samples occurs slowly enough for complexation of the ligand and ligand analog in the receptor to occur during spreading. The receptor may be immobilized in the spreading layer. The beads are generally present in a wide variety of sizes from 1 to 200 µm, although large beads in the range of 20 to 40 µm predominate. Such a spreading layer is useful for a variety of different analytes. This is because spreading requirements are similar.

The problem is that such beads are not always adequate in immunoassays. Different ligands require different concentrations of receptors. Very large beads may not provide sufficient surface area in all cases to accommodate the required receptor concentration. Moreover, the beads generally used in spreading layers do not have the requisite surface quality and properties to which biologically active materials, such as antibodies, can be conveniently attached. For example, large beads do not generally contain reactive functional groups for the covalent immobilization of biologically active species such as antibodies.

Even when the large beads are prepared with reactive groups for covalent bonding they are not as good for immobilization of antibodies as small beads because of the method of their preparation. Spreading layer beads are generally prepared by suspension polymerization using limited coalescence. This preparation has a negative effect on immobilization of receptors and ligands. This method of preparation leaves various residual materials, such as surfactants and silica stabilizer on the bead surfaces. The residual materials inhibit the attachment of receptors to the beads. These residual materials can also mask the presence of attached receptors. The masked receptors are then unable to immobilize ligands.

The present invention solves the foregoing problem by providing an analytical element for immunoassays comprising a support and a spreading layer characterized in that the element contains,

a) a first population of relatively large polymeric beads having a diameter in the range of 10 to 200 µm, preferably 20 to 40 µm, to facilitate uniform spreading of liquids applied to the element; and

b) a second population of relatively small polymeric beads having (i) a diameter in the range of 0.1 to 5 µm; (ii) receptors covalently bound to the bead surface through surface reactive groups; and (iii) surfaces which are free of residual materials, such as surfactants and collidal silica stabilizer.

For convenience, the first population of polymer beads will be referred to as large beads and the second population will be referred to as small beads.

Preferably, the polymeric beads of the second population are monodispersed. Monodispersed means the distribution of particle sizes of 3 $\sigma$ (sigma) is equal to or less than 7% of the mean particle diameter. The larger particles of the spreading layer are typically polydispersed (first population) having widely varying sizes.

The present invention also provides a method for the assay of an immunologically reactive ligand in a liquid using the above element, comprising the steps of:

A. in the presence of a labeled ligand analog, contacting a finite area of the spreading layer with a sample of the liquid to form an immobilized ligand-receptor complex within the finite area and to effect substantially horizontal separation of uncomplexed ligand from the immobilized complex during liquid spreading, and

B. after the completion of step A, determining the immobilized complex in the center of the finite area.

The present invention also provides a method for assaying an immunological reactive ligand in a liquid with an analytical element described above comprising the steps of:

A. in the presence of a labeled ligand analog, contacting a finite area of the spreading layer with a sample of the liquid to form an immobilized ligand-receptor complex within the finite area;

B. adding a wash solution to effect substantial horizontal separation of uncomplexed ligand from the immobilized complex; and

C. after the completion of step B, determining the immobilized complex in the center of the finite area.

The present invention provides a greatly improved element for immunoassays. The small polymer beads provide the surface area necessary to accommodate the concentration of receptor required for any immunoassay. The beads are prepared from polymers that provide the surface reactive functional groups required to immobilize receptors such as antibodies. Moreover, the beads are free of materials such as surfactants and emulsifiers that inhibit the attachment of receptors and also mask receptors.

The element and the assay can be used in highly automated analyzers.

The elements provided by the present invention are useful in any immunoassay, including assays using a sandwich technique described in Immuno Chemical Techniques, pp. 206-207. However, for purposes of brevity, utility will be illustrated herein in the context of a competitive immunoassay. In the latter assay, the species to be determined and a corresponding labeled species compete for a fixed amount of a common reactant. The species to be determined is referred to herein as a ligand, and the labeled species is referred to as a ligand analog. Compounds which specifically recognize the ligand and ligand analog and react to form complexes with them are referred to herein as receptors. The receptor and the ligand analog form a conjugate pair. Any member of the pair can function as a receptor or a ligand.

The element can be used to determine low concentrations of immunologically reactive ligands in a liquid, such as a biological fluid (for example, whole blood, serum, plasma, urine, spinal fluid, suspensions of human or animal tissue, feces, saliva, lymphatic fluid and the like). The ligands can be determined at concentrations as low as $10^{-15}$ molar, and most generally at a concentration of from $10^{-10}$ to $10^{-4}$ molar.

Ligands which can be so determined, either quantitatively or qualitatively, include therapeutic drugs (for example, phenobarbital, theophylline, gentamicin, quinidine, phenytoin, propanolol, carbamazepine, tobramycin, lidocaine, procainamide and the like), natural or synthetic steroids (for example, cortisol, aldosterone, testosterone, progesterone, estriol, etc.), hormones (for example, thyroid hormones, peptide hormones, insulin, etc.), proteins (for example, albumin, IgG, IgM, ferritin, C-reactive protein, isoenzymes, apolipoproteins, etc.), antigens, antibodies including monoclonal antibodies, and other species which will naturally react with a receptor. This invention is particularly useful for the determination of therapeutic drugs, such as digoxin, phenytoin, theophylline, or phenobarbital and hormones such as thyroxine or triiodothyronine.

Biological receptors of interest having the requisite free amino or sulfhydryl group for covalent bonding to the small polymer beads include:

a) Protein A or protein G which has an affinity for the Fc portion of IgG antibodies.

b) Avidin or avidin complexes which have an affinity for biotin or biotin complexes.

Avidin and biotin derivatives which can be used to prepare the reagents of this invention include streptavidin, succinylated avidin, monomeric avidin, biocytin (that is, biotin-ε-N-lysine), biocytin hydrazide, amine or sulfhydryl derivatives of 2-iminobiotin and biotinyl-ε-aminocaproic acid hydrazide,

biotin derivatives, such as biotin-N-hydroxysuccinimide ester, biotinyl-ε-aminocaproic acid-N-hydroxysuccinimide ester, sulfosuccinimidyl 6-(biotin amido)hexanoate, N-hydroxysuccinimideiminobiotin, biotinbromoacetylhydrazide, p-diazobenzoyl biocytin and 3-(N-maleimidopropionyl)biocytin.

c) Monoclonal antibodies which have specialized affinity for the antigen against which it was raised and their antigens.

d) Polyclonal antibodies and their respective antigens.

e) Lysine which has an affinity for plasminogen.

f) Proteins and other biological macromolecules which have specialized affinity for another protein or biological macromolecule of interest such as gelatin which has affinity for fibronectin.

g) Small molecule and oligomeric species having specialized affinity for oligomeric or macromolecular biological molecules, for example, sugars, drugs, DNA bases, DNA oligomers and hormones.

h) Macromolecules that have specificity for particular classes of biological molecules such as Concanavalin A which has specificity for certain sugars and sugar-containing macromolecules; heparin which has affinity for coagulation factors, lipoproteins, plasma proteins, etc.

i) Small molecules that have specialized affinity for classes of biological molecules such as the dye Cibarcon$^R$ Blue F3G-A and other protein specific hydrophobic dyes that have specificity for albumen, enzymes requiring adenyl-containing cofactors, coagulation factors and interferron.

It will be clear to those skilled in the art that, depending upon the objective of the assay, ligands can be receptors, and receptors can be ligands. For example, a ligand comprising phenobarbital can be attached to the small beads when it is desired to assay for a phenobarbital antibody or for phenobarbital using a labeled phenobarbital antibody.

As already stated, the small polymeric beads have receptors on their surface. The receptors are bonded

to the beads through surface reactive groups which are directly or indirectly reactive with free amino groups, sulfhydryl groups, carboxy groups, aldehyde or ketone groups of the receptors.

Useful surface reactive groups include:

a) active halogen groups;
b) activated 2-substituted ethylsulfonyl or activated vinylsulfonyl groups;
c) reactive carboxyl groups;
d) epoxy groups;
e) isocyanate groups;
f) aziridine groups;
g) aldehyde groups;
h) 2-substituted ethylcarbonyl groups; and
i) succinimidoxycarbonyl groups.

Surface reactive groups a), b), c) and i) are preferred.

It will be clear to those skilled in the art that, depending upon the objectives and design of a particular assay, the surface active groups a)-i) can be part of the receptor, then the amino, sulfhydryl, carboxy and aldehyde groups would serve as surface active groups through which the receptor is attached to the small beads.

In general, the polymers employed to form the small polymer beads conform to the general structure:

$$-(A)_o-(B)_p-(D)_q- \qquad I$$

wherein -A- represents recurring units derived from one or more hydrophobic ethylenically unsaturated monomers,

-B- represents recurring units derived from one or more ethylenically unsaturated monomers having the requisite reactive groups which will directly or indirectly react with the free amine or sulfhydryl groups of the receptors; and

-D- represents recurring units derived from one or more ethylenically unsaturated monomers which are different than those represented by -A- or -B-.

In formula I, o is from 0 to 99.9 mole percent, p is from 0.1 to 100 mole percent, and q is from 0 to 20 mole percent. Preferably, o is from 45 to 99 mole percent, p is from 1 to 50 mole percent, and q is from 0 to 10 mole percent.

The -A- recurring units are derived from one or more hydrophobic ethylenically unsaturated monomers. Such monomers are insoluble in water. Representative hydrophobic monomers include, but are not limited to, styrene and styrene derivatives (for example, vinyltoluene, 2,5-dimethylstyrene, 4-t-butylstyrene and 2-chlorostyrene), acrylic and methacrylic acid esters (for example, n-butyl acrylate, propyl methacrylate, methyl acrylate, ethyl methacrylate, 2-ethylhexyl methacrylate, N-phenylacrylamide and methyl methacrylate), acrylonitrile and vinyl acetate.

The polymer can be crosslinked, if desired, in any suitable fashion. One method is to incorporate a small amount, that is up to 15 mole percent, and preferably from 0.3 to 5 mole percent, of a monomer having two or more ethylenically unsaturated polymerizable groups. These monomers are included among the hydrophobic monomers from which A is derived. Representative monomers are described in Research Disclosure, publication 19551, July, 1980, page 304, and include for example, divinylbenzene, ethylene dimethacrylate, N,N′-methylenebisacrylamide, 2,2-dimethyl-1,3-propylene diacrylate, allyl acrylate, ethylidyne trimethacrylate and ethylene diacrylate.

Particularly useful monomers from which -A- is derived are styrene, vinyltoluene, ethylene dimethacrylate, butyl acrylate, divinylbenzene, 2-ethylhexyl methacrylate and methyl methacrylate.

The -B- recurring units comprise an appended surface reactive group that readily reacts with an amine or sulfhydryl group with or without the use of an intermediate crosslinking agent. The B groups can therefore be derived from any monomer containing such reactive groups. Preferred monomers are those comprising appended electrophilic surface reactive groups a) to i) described, supra.

One preferred class of monomers which provide the requisite reactive groups are those comprising an active halogen atom which readily reacts with amine and sulfhydryl groups.

Examples of monomers having an active halogen atom include vinyl chloroacetate, vinyl bromoacetate, haloalkylated vinyl aromatics (for example, chloromethylstyrene or bromomethylstyrene), haloalkyl acrylic or methacrylic esters (for example, chloroethyl methacrylate, 3-chloro-2-hydroxypropyl methacrylate and 3-chloropropyl acrylate) and others known to one skilled in the art. The haloalkylated vinyl aromatics, for example, those having active haloalkyl groups of 1 to 3 carbon atoms, are preferred when the active halogen atom is used as the reactive group. Chloromethylstyrene is very useful.

Although monomers having active halogen atoms exhibit many advantages, monomers having activated 2-substituted ethylsulfonyl and vinylsulfonyl groups possess additional advantages in that proteins can be attached to the polymers under milder conditions and require less process control during manufacture. This renders manufacture more efficient and less costly. A number of representative monomers having the latter groups are known in the art, including those disclosed in U.S. Patents 4,161,407 and 4,548,870.

Preferred activated 2-substituted ethylsulfonyl and vinylsulfonyl monomers can be represented by the formula (II):

$$CH_2{=}\overset{\displaystyle R}{\overset{|}{C}} - L - \overset{\displaystyle O}{\overset{\|}{\underset{\underset{\displaystyle O}{\|}}{S}}} - R^1 \qquad (II)$$

wherein R is hydrogen or substituted or unsubstituted alkyl (generally of 1 to 6 carbon atoms, such as methyl, ethyl, isopropyl or hexyl). Preferably, R is hydrogen or methyl.

$R^1$ is $-CH{=}CHR^2$ or $-CH_2CH_2X$ wherein X is a leaving group which is displaced by a nucleophile or is eliminated in the form of HX by treatment with a base (such as halo, acetoxy, alkylsulfonyloxy such as methylsulfonyloxy, arylsulfonyloxy such as p-tolylsulfonyloxy, trialkylammonio, for example, a trimethylammonio salt or pyridinio salt). $R^2$ is hydrogen, substituted or unsubstituted alkyl (generally of 1 to 6 carbon atoms as defined for R), or substituted or unsubstituted aryl (generally of 6 to 12 nuclear carbon atoms, such as phenyl, naphthyl, xylyl or tolyl). Preferably, $R^1$ is $-CH_2CH_2X$.
This group, which is an activated 2-substituted ethyl group, can be substituted with any group which does not impair the displacement of the leaving group X.

L is a linking group which can be a substituted or unsubstituted alkylene generally having 1 to 20 carbon and hetero atoms in the backbone. This definition of alkylene is meant to include alkylene groups interrupted or terminated with oxy, thio, $-NR^3-$ [wherein $R^3$ is hydrogen, substituted or unsubstituted alkyl of 1 to 6 carbon atoms (such as methyl, chloromethyl or 2-hydroxyethyl) or substituted or unsubstituted aryl of 6 to 10 carbon atoms (such as phenyl, naphthyl or xylyl)], ester (-COO-), amide (-CONH-), urylene

$$(-NH\overset{\displaystyle O}{\overset{\|}{C}}NH-),$$

sulfonyl ($-SO_2-$), carbonate, sulfonamide, azo, phosphono or other similar groups. Representative alkylene groups include methylene, ethylene, isobutylene, hexamethylene, carbonyloxyethoxycarbonyl, methylenebis(iminocarbonyl), carbonyloxydodecylenecarbonyloxyethylene, carbonyliminomethyleneiminocarbonyliminoethylene, carbonyliminomethyleneiminocarbonylethylene and other groups described or suggested by U.S. Patents 4,161,407 and 4,548,870, noted above.

L can also be substituted or unsubstituted arylene generally having 6 to 12 nuclear carbon atoms. Representative arylene groups include phenylene, tolylene, naphthylene and others noted in the patents mentioned above. Also included in this definition of L are divalent groups which are combinations of one or more of each of the alkylene and arylene groups defined above (for example, arylenealkylene, alkylenearylenealkylene and others readily determined by one of ordinary skill in the art). Preferably, L is substituted or unsubstituted phenylenealkylene, phenylenealkylene substituted with one or more alkyl groups (as defined for R), alkoxy groups (generally of 1 to 6 carbon atoms, for example, methoxy, propoxy or butoxy) or halo groups, or carbonyliminomethyleneiminocarbonylethylene.

Representative 2-substituted ethylsulfonyl and vinyl sulfonyl monomers from which B can be derived include m & p-(2-chloroethylsulfonylmethyl)styrene, m & p-[2-(p-tolylsulfonyloxy)ethylsulfonylmethyl]styrene, m & p-vinylsulfonylmethylstyrene, N-[m & p-(2-chloroethylsulfonylmethyl)phenyl]acrylamide, and N-[2-(2-chloroethylsulfonyl)ethylformamidomethyl]acrylamide. The first monomer is preferred.

Another preferred reactive group that can be appended to form recurring units B is the carboxyl group.

Carboxyl groups can be added to the particles by incorporating monomers containing such groups as, for example, acrylic acid, methacrylic acid, itaconic acid, 2-carboxyethyl acrylate, fumaric acid, maleic acid, 2-carboxyethyl methacrylate carboxymethylstryene, methacrylamidohexanoic acid, N-(2-carboxy-1,1-dimethylethyl)acylamide, and the like), or by further chemical reaction of a polymer having other reactive groups which can be converted to carboxyl groups (for example, by hydrolysis of anhydrides, such as maleic anhydride, or by oxidation of surface methylol or aldehyde end groups).

An auxiliary crosslinking agent is used to covalently attach proteins, for example, antigens, antibodies, haptens, etc. via the carboxyl groups since the carboxy groups alone react too slowly with amine and sulfhydryl

groups for most practical purposes. One useful class of auxiliary crosslinking agents are the well-known carbodiimides, for example, 1-cyclohexyl-3-[2-morpholinyl-4-ethyl]carbodiimide metho-p-toluenesulfonate, which have been used for crosslinking gelatin in photographic gelatin layers and for making diagnostic reagents as described in U.S. Patent 4,181,636.

Another preferred class of auxiliary crosslinking agents includes the carbamoylonium salts such as are described in U.S. Patent 4,421,847. Representative carbamoylonium compounds include 1-(4-morpholinocarbonyl)-4-(2-sulfoethyl)pyridinium hydroxide, inner salt, and 1-(4-morpholinocarbonyl)-pyridinium chloride.

Other monomers which can be incorporated in the polymers to provide the requisite reactive groups include monomers containing epoxy groups (such as glycidyl acrylate, glycidyl methacrylate, vinyl glycidyl ether or methallyl glycidyl ether), monomers containing isocyanate groups (such as isocyanatoethyl acrylate, isocyanatoethyl methacrylate, or α, α-dimethylmetaisopropenylbenzyl isocyanate), monomers containing an aziridine group [such as vinylcarbamoyl aziridine, N-methacryloylaziridine, N-acryloylaziridine and 2-(1-aziridinyl)ethyl acrylate], monomers containing aldehyde groups (such as vinyl benzaldehyde or acrolein) or 2-substituted ethylcarbonyl containing monomers (such as 2-chloroethyl acrylate, 2-chloroethyl methacrylate, 2-methylsulfonyloxyethyl methacrylate and 2-p-tolysulfonyloxyethyl acrylate).

D represents recurring units derived from one or more ethylenically unsaturated monomers other than those represented by A or B. Such monomers can have ionic or other hydrophilic groups which add dispersion stability to the resulting particles in aqueous solution or influence the biological activity of an immobilized ligand. Useful ionic monomers include, but are not limited to, sodium 2-acrylamido-2-methylpropanesulfonate, sodium 3-acryloyloxypropanesulfonate, sodium acrylate, sodium methacrylate, and sodium styrenesulfonate, as well as other known sulfonates, sulfates, carboxylates, their salts or anhydrides, and useful nonionic polar monomers include 2-hydroxyethyl acrylate, 2,3-dihydroxypropyl acrylate, acrylamide, 2-hydroxyethyl methacrylate, N-isopropylacrylamide, 2-hydroxypropyl methacrylate, acrylonitrile and N-isobutoxymethyl acrylamide. Preferred monomers are sodium 2-acrylamido-2-methylpropanesulfonate, sodium acrylate, sodium 3-acryloyloxypropanesulfonate, sodium methacrylate, 2-hydroxyethyl acrylate, 2,3-dihydroxypropyl acrylate, acrylamide, N-isopropylacrylamide and acrylonitrile.

The small polymer beads can be homogeneous particles being composed of the same polymer throughout, or they can be particles composed of more than one polymer such as graft copolymers as described, for example, in U.S. Patent 3,700,609 and core-shell polymers described for example, in U.S. Patent 4,401,765. This is advantageous when any of the recurring units of the polymer that must be on the particle surface such as those containing the reactive groups or groups that impart dispersion stability are expensive. A polymer particle can be prepared from relatively inexpensive monomers, or monomers that regulate buoyancy, then polymerization is continued to add a shell of a different polymer having the requisite surface groups.

The small polymer beads can be prepared using emulsion polymerization techniques (including batch, semi-continuous and continuous). Emulsion polymerization is preferred as it can be used to provide generally smaller particles without the use of surfactants or emulsifiers as described for example, in U.S. Patent 4,415,700 (noted above) and Research Disclosure publication 15963 (July, 1977). Research Disclosure is a publication available from Kenneth Mason Publications, Ltd., The Old Harbourmaster's, 8 North Street, Emsworth, Hampshire P010 7DD, England.

Staged emulsion polymerization can be used to provide a core-shell polymer composed of two different polymers. Emulsion polymerization of the core is carried to substantial completion by continuously adding reactants to a reaction vessel under standard conditions. Monomers and catalysts needed to make the shell polymer are then continuously added to the vessel containing the latex of the core polymer. In this manner, the shell has a definite known composition rather than being a mixture of core and shell monomers.

Representative polymers useful in this invention include the following: poly(m & p-chloromethylstyrene), poly(styrene-co-m & p-chloromethylstyrene-co-2-hydroxyethyl acrylate) (67:30:3 molar ratio), poly(styrene-co-m & p-chloroethylsulfonylmethylstyrene) (95.5:4.5 molar ratio), poly{styrene-co-N-[m & p-(2-chloroethylsulfonylmethyl)phenyl]acrylamide} (99.3:0.7 molar ratio), poly(m & p-chloromethylstyrene-co-methacrylic acid)(95:5, 98:2 and 99.8:0.2 molar ratio), poly(styrene-co-m & p-chloroethylsulfonylmethylstyrene-co-methacrylic acid)(93.5:4.5:2 molar ratio), poly{styrene-co-N-[m & p-(2-chloroethylsulfonylmethyl)phenyl]acrylamide-co-methacrylic acid}(97.3:0.7:2 molar ratio), poly(styrene-co-m & p-chloromethylstyrene)(70:30 molar ratio), poly(styrene-co-vinylbenzyl chloride-co-acrylic acid) (85:10:5 molar ratio), poly(styrene-co-acrylic acid) (99:1 molar ratio), poly(styrene-co-methacrylic acid) (90:10 molar ratio), poly(styrene-co-acrylic acid-co-m&p-divinylbenzene) (89:10:1 molar ratio), poly(styrene-co-2-carboxyethyl acrylate) (90:10 molar ratio), poly(methyl methacrylate-co-acrylic acid) (70:30 molar ratio), poly(styrene-co-m & p-vinylbenzaldehyde)(95:5 molar ratio), and poly(styrene-co-m & p-vinylbenzaldehyde-co-methacrylic acid)(93:5:2 molar ratio).

The receptors are covalently bonded to the particles through the surface reactive groups a) through i) referred to hereinbefore. The groups are directly or indirectly reactive with nucleophilic free amino groups and

sulfhydryl groups of the biological receptors.

In cases where the biological species is a protein that contains, or has been modified to contain, electrophilic groups (carboxy, aldehyde, etc.) the polymer particles can have surface reactive nucleophilic groups such as amines, sulfhydryl, etc.

A general procedure for attaching receptors to the small polymer beads includes covalently attaching the selected receptor to the beads using generally known reactions. With many pendant groups, for example, the haloalkyl, 2-substituted activated ethylsulfonyl and vinylsulfonyl, the receptor can be directly attached to the beads. Generally, the beads are mined with the receptor in an aqueous buffered solution (pH generally from 5 to 10) and a concentration of from 0.1 to 40 weight percent polymer particles (preferably from 0.1 to 10 weight percent). The amount of receptor is at a ratio to polymer of from 0.1:1000 to 1:10, and preferably from 1:100 to 1:10. Mixing is carried out at a temperature in the range of from 5 to 50°C, and preferably at from 5 to 40°C, for from 0.5 to 48 hours. Any suitable buffer can be used.

In some instances, the pendant reactive groups on the outer surface must be modified or activated in order to cause covalent attachment of the ligand. For example, carboxyl groups must be activated using known carbodiimide or carbamoylonium chemistry, described supra.

In other instances, an epoxy group on the outer surface can be hydrolyzed to form a diol compound capable of reacting with cyanogen bromide which can act as a coupling agent for amine groups in the immunological species. Aldehydes can react directly with amines to form a Schiff's base which can be subsequently reduced to form a covalent link. Alternatively, the aldehyde can be oxidized to an acid and chemistry identified above for carboxyl groups can be used to form an amide linkage.

Any reactive amine- or sulfhydryl-containing receptor can be attached to the monodispersed polymeric beads as long as that receptor contains a reactive amine or sulfhydryl group, respectively which will react with the reactive groups on the polymer or with the intermediate formed by the reaction of a carbodiimide or a carbamoylonium compound with carboxyl groups on the particles in the case which the polymer has reactive carboxyl groups.

The small polymer beads having reactive groups that readily react directly with the amine or sulfhydryl groups on the receptors are simply mixed with the receptors, in an appropriate buffer if necessary, and allowed to react.

The attachment of the receptor to carboxyl group-containing monodispersed polymer beads, however, is carried out in two steps, the first of which involves contacting an aqueous suspension of the particles with a carbodiimide or a carbamoylonium compound to produce reactive intermediate polymer particles having intermediate reactive groups in place of the carboxyl groups. This step is carried out at a suitable pH using suitable acids or buffers to provide the desired pH. Generally, the pH is less than 6, but this is not critical as long as the reaction can proceed. More likely, the pH is between 3.5 and 7. The molar ratio of carbodiimide or carbamoylonium compound to the carboxyl groups on the surface of the particles is from 10:1 to 500:1.

In the second step of the method, the reactive intermediate formed in the first step is contacted with a reactive amine- or sulfhydryl-group containing receptor. A covalent linkage is thereby formed between the particles and the receptor. The weight ratio of the receptor to the polymeric particles is generally from 1:1000 to 1:1, and preferably from 1:100 to 1:10.

The porous spreading layer has suitable porosity for accommodating a test sample (for example, 1 to 100 $\mu\ell$), diluted or undiluted. Preferably, the spreading layer is isotropically porous, which property is created by interconnected spaces between the particles comprising the Zone. By isotropically porous is meant that the spreading layer uniformly spreads the applied fluid radially throughout the layer.

Useful spreading layers are disclosed in U.S. Patents 4,670,381; 4,258,001 and 4,430,436. Particularly useful spreading layers are those having a particulate structure formed by organo-polymeric particles and a polymeric adhesive for those particles described in U.S. Patent 4,258,001. Maintaining particulate integrity of the organ-polymeric particles in the particulate structure with the polymeric adhesive prevents the coalescence and flow of the particles into the voids, and the concentration of adhesive at those particle surface areas of the structure which are contiguous to adjacent particles insures that the adhesive does not flow into and clog the voids. The thickness of the described particulate structure can be varied depending upon the size of the organo-polymeric particles. For optimum liquid spreading, the particle coverage is generally within the range of from 25 to 200 $g/m^2$.

The heat-stable, organo-polymeric particles useful in the spreading layer are generally spherical beads having a particle size in the range of from 20 to 40 $\mu m$ in diameter. These are the large beads of the first population of polymeric beads.

The particles can be composed of a wide variety of organic polymers, including both natural and synthetic polymers, having the requisite properties. Preferably, however, they are composed of one or more addition polymers described in the aforementioned patents.

Particularly useful addition polymers include those listed in Table I of U.S. Patent 4,258,001. The bracketed numbers in the table represent the weight ratio of monomers in the monomer blend used to prepare the polymer. Poly(vinyltoluene-co-p-t-butylstyrene-co-methacrylic acid) [61:37:2], poly(styrene-co-n-butyl acrylate) [75:25] and polystyrene are preferred polymers. The organo-polymeric particles can contain other addenda, if desired, as known in the art.

The polymeric adhesive that is useful in this invention bonds the organo-polymeric particles to one another to provide a coherent, three-dimensional lattice in the spreading layer. The details of this adhesive are also provided in U.S. Patent 4,258,001. Generally, the adhesive is composed of an organic polymer different from the specific polymer contained in the particles, although quite commonly the adhesive represents a polymer containing many repeating units which are identical or similar to some of those present in the polymer composition of the particles.

Particularly useful addition polymers include those listed in Table II of U.S. Patent 4,258,001 and in U.S. Patent 4,283,491. The bracketed numbers in the table represent the weight ratio of monomers in the monomer blend used to prepare the polymer. Poly(methyl acrylate-co-2-acetoacetoxyethyl methacrylate-co-2-acrylamido-2-methylpropanesulfonic acid) [88:7:5]; poly(N-vinyl-2-pyrrolidone); poly(n-butyl acrylate-co-styrene-co-2-acrylamido-2-methylpropane sulfonic acid, sodium salt) [75:20:5]; and poly(methyl acrylate-co-2-acryl-amido-2-methylpropane sulfonic acid, sodium salt-co-acetoacetoxy ethyl methacrylate) [95:2:3] are preferred adhesive polymers.

Methods for preparing the particulate structure with the above-described particles and adhesives are provided in the above noted patents.

The spreading layer of the element is carried on a suitable support. Such a support can be any suitable dimensionally stable, and preferably, nonporous and transparent (that is, radiation transmissive) material which transmits electromagnetic radiation of a wavelength between 200 and 900 nm. A support of choice for a particular element should be compatible with the intended mode of detection (reflection, transmission or fluorescence spectroscopy). Useful support materials include polystyrene, polyesters [for example, poly(ethylene terephthalate)], polycarbonates, cellulose esters (for example, cellulose acetate), etc.

The element can comprise one or more layers, for example, separate or combined reagent/spreading layer and a gelatin buffer layer containing other necessary additives, coupling enzymes, etc. The large and small polymeric beads can either be coated in the same or different layers. The small beads can be coated before, concurrently with or after the large beads.

The reagent layer or the spreading layer of the element can contain the indicator composition comprising one or more reagents dispersed in one or more synthetic or natural binder materials, such as gelatin, or other naturally-occurring colloids, homopolymers and copolymers, such as poly(acrylamide), poly(vinyl pyrrolidone), poly(N-isopropylacrylamide), poly(acrylamide-co-N-vinyl-2-pyrrolidone) and similar copolymers.

Other optional layers, for example, subbing layers, radiation-blocking layers, etc. can be included if desired. All layers of the element are in fluid contact with each other, meaning that fluids and reagents and uncomplexed reaction products in the fluids can pass between superposed regions of adjacent layers.

Additional layers can also be used, such as subbing layers, interlayers, etc. These layers can also contain reagents for the assays, if desired.

The assay can be carried out using any suitable label which can be attached to the ligand to form a ligand analog. Useful labels include radioactive tags, dyes, fluorescers, enzymes, enzyme substrates, enzyme inhibitors, allosteric effectors, cofactors and other known enzyme modulators. Enzymes, such as glucose oxidase, peroxidase, alkaline phosphatase and galactosidase are preferred labels.

When an enzyme label is used, the substrate for the enzyme is present in the element or added thereto in the wash liquid. The substrate can be added to the element prior to or simultaneously with the liquid sample, or after completion of the binding reaction. It is within the skill of the ordinary worker in clinical chemistry to determine a suitable substrate for a given label. The substrate can be a material which is directly acted upon by the enzyme label, or a material that is involved in a series of reactions which involve enzymatic reaction of the label. For example, if the enzyme label is peroxidase, the substrate is hydrogen peroxide. Using glucose oxidase as an example, the substrate glucose is generally present in the reagent layer or added in the wash liquid to yield 0.01 moles/$m^2$, and preferably from 0.001 to 0.1 mole/$m^2$. A worker skilled in the art would know how to adjust the amount of a particular substrate for the amount of enzyme label used in the assay.

When certain labels are used, for example, enzymes, cofactors, enzyme substrates or enzyme modulators, the reagent layer contains an indicator composition comprising one or more reagents which provide a detectable species as a result of reaction of the label. Preferably, the indicator composition is a colorimetric indicator composition which provides a colorimetrically detectable species as a result of enzymatic reaction of an enzyme-labeled ligand analog with a substrate.

The indicator composition can be a single compound which produces a detectable dye upon enzymatic

reaction, or a combination of reagents which produce the dye. For example, when glucose is used as the substrate and glucose oxidase as the enzyme label, the colorimetric indicator composition can include a coupler and oxidizable compound which react to provide a dye. Alternatively, the composition can include a leuco dye and peroxidase or another suitable peroxidative compound which generate a detectable dye as a result of the formation of hydrogen peroxide produced when glucose oxidase converts glucose to gluconic acid. Useful leuco dyes are known in the art and include those, for example, described in U.S. Patent 4,089,747 and EP Publication 0162685. The particular amounts of the colorimetric indicator composition and its various components are within the skill of a worker in the art.

The layers of the element can contain a variety of other desirable but optional components, including surfactants, thickeners, buffers, hardeners, antioxidants, coupler solvents, and other materials known in the art. The amounts of these components are also within the skill of a worker in the art.

The ligand analogs useful in the practice of this invention can be prepared using known starting materials and procedures, or obtained commercially. Generally, the ligand moiety of the analog is attached to the label (for example, an enzyme moiety or fluorescer) through a covalent bond.

The immunoassay can be manual or automated. In general, the amount of a ligand in a liquid is determined by taking the element from a supply roll, chip packet or other source and physically contacting a finite area of the spreading layer with a sample of the liquid, 1 to 100 μl. The finite area which is contacted is generally no more than 100 $mm^2$.

If the ligand analog is not incorporated in the element during manufacture, it can be mixed with the test sample simultaneously with or prior to contact with the element.

After sample application in either embodiment, the element is exposed to any conditioning, such as incubation, heating or the like, that may be desirable to quicken or otherwise facilitate obtaining the test result.

The amount of ligand is determined by passing the element through a suitable apparatus for detecting the complexed ligand analog directly or the detectable species formed as a result of enzymatic reaction of an enzyme label and a substrate. For example, the species can be detected with suitable radiometric, fluorometric or spectrophotometric apparatus using generally known procedures. In an enzymatic reaction, the resulting product is determined by measuring, for example, the reflection or transmission density or fluorescence in the center of the finite area which was contacted with the test sample. The area which is measured is generally from 3 to 5 mm in diameter for competing assays. The amount of ligand in the liquid sample is inversely proportional to the amount of label measured in the center of the finite area. In a preferred embodiment a separate wash step is required in order to separate complexed ligand from uncomplexed ligand. Generally, label measurement is carried out after from 5 to 180 seconds after sample contact and spreading or application of the wash liquid.

The following examples establish the utility of the present invention. In these examples core/shell mono-dispersed polymer beads containing polystyrene copolymerized with various other monomers, such as m+p-vinylbenzylchloride, m+p-(2-chloroethylsulfonylmethyl)styrene, acrylic acid, methacrylic acid, o,m+p-divinylbenzenes and ethylene glycol dimethacrylate. Specific mono-dispersed beads with their mole percent compositions were:

1. A core comprising poly(styrene-co,-o,m+p-divinylbenzenes) (99.4:0.6), and a shell comprising poly(styrene-co-m+p-)2-chloroethylsulfonylmethyl-styrene-co-o,m+p-divinylbenzene), (99.8:1.2); useful for digoxin and phenytoin.
2. A core comprising poly(styrene-co-ethylene glycol dimethacrylate) (99:1), and a shell comprising poly(styrene-co-m+p-(2-chloroethylsulfonylmethyl)-styrene-co-ethylene glycol dimethacrylate) (93.5/4.5/1); useful for digoxin and thyroxine.

The larger spreading layer beads were poly(m+p-vinyltoluene-co-methacrylic acid). The size range was from about 20μm to about 40μm.

The adhesive was poly(methyl acrylate-co-2-acrylamido-2-methylpropane sulfonic acid, sodium salt-co-2-acetoacetoxyethyl methacrylate) (94.9:2.1:3).

## Materials

| | |
|---|---|
| DMSO | (Dimethyl sulfoxide) |
| TEA | (Triethanolamine buffer) |
| MOPS | (3-[N-morpholino]propanesulfonic acid buffer) |
| ALP | (alkaline phosphatase) |
| ANS | (8-anilino-1-naphthalene sulfonic acid) |

The wash solution for thyroxine and phenytoin comprises p-nitrophenyl phosphate substrate (15mM) in 2-amino-2-methyl-1-propanol (1.5M, pH 10.3).

Thyroxine - ALP conjugate was prepared by the procedure of M. Ito et al, Clin. Chem., 30, pp. 1682-1685 (1984).

Phenytoin - ALP conjugate was prepared by the procedure of B.F. Erlanger et al, J. Biol. Chem., 234, p. 1090 (1959), using 5,5-diphenylhydantoin-3-(omega-valeric acid).

Other materials were:

Zonyl™ FSN surfactant (DuPont, Wilmington, Delaware U.S.A.);

Triton™ X-100 surfactant (Rohm & Haas, Philadelphia, Pennsylvania, U.S.A.); and

the remainder from Eastman Kodak Co. (Rochester, New York, U.S.A.), or prepared using known starting materials and procedures.

As used in the context of this disclosure and the claims, I.U. represents the International Unit for enzyme activity defined as one I.U. being the amount of enzyme activity required to catalyze the conversion of 1 μmole of substrate per minute under standard pH and temperature conditions for the enzyme.

The water soluble polymer layer in the element is comprised of any water soluble polymer. Preferred polymers are polyvinyl alcohol, polyvinylpyrrolidone, polyacrylamide, poly(acrylamide-co-N-vinyl-2-pyrrolidone) or gelatin, or any combination of these polymers.

Example 1 - Assay for Thyroxine ($T_4$)

This example illustrates a competitive immunoassay for $T_4$ using a three-layer format and a separate wash step. The following element structure is useful for assaying T4.

| | | Coverage ($g/m^2$) | |
|---|---|---|---|
| | | Preferred | Ranges |
| Spreading Layer | Small Beads Containing T4 Antibodies as Receptors | 0.03 | 0.005-5.00 |
| | Large Beads | 130 | 25-200 |
| | Polyvinylpyrrolidone | 0.08 | 0.02-0.2 |
| | TX-100 | 0.05 | 0.01-0.2 |
| | Adhesive | 2 | 1-15 |
| Subbing Layer | Acrylamide-co-N-vinyl-2-pyrolidone (50:50) | 1 | 0.2-5 |
| | TEA (pH 7.5) | 0.15 | 0.01-1 |
| | BSA | 0.05 | 0.01-0.2 |
| | TX-100 | 0.01 | 0.005-0.05 |
| | MgCl2 | 0.02 | 0.01-0.05 |
| | ZnCl2 | 0.001 | 0.0005-0.005 |
| Gelatin Layer | Hardened Gelatin | 10 | 5-20 |
| | TX-100 | 0.02 | 0.01-1.0 |
| | TEA, pH 7.5 | 0.7 | 0.1-2.0 |

A series of $T_4$ standards varying in concentration from 2 x $10^{-5}$M to 2 x $10^{-10}$M were prepared in the buffer described below from a 1 x $10^{-3}$M $T_4$ stock solution in DMSO. The buffer consisted of phosphate buffed saline, pH 7.4, and bovine serum albumin (1%) and ANS (8.7 x $10^{-4}$M). A $T_4$-ALP conjugate solution was prepared at a concentration of a 1.0 x $10^{-8}$M. The $T_4$ solutions and $T_4$-ALP solution were mixed, 1 to 1, so that the final concentration of $T_4$-ALP was 5 x $10^{-9}$M.

Ten microliter aliquots were spotted onto the $T_4$ element. After 5 minutes at 37°C, a wash solution (10µL) was added to wash unbound $T_4$-ALP away from the center of the element. After one minute, the $\triangle D_R$ was measured for 30 seconds in the center of the element at 37°C and 400 nm. The Williams-Clapper transform [J. Optical Soc. Am., 43, p. 595 (1953)] was used to convert the $D_T$ values. Results are shown in Table 2.

Table 2

| $T_4$ Conc | Rate ($\Delta D_T$/min) |
|---|---|
| $1 \times 10^{-10}$M | 0.0289 |
| $1 \times 10^{-9}$M | 0.0293 |
| $1 \times 10^{-8}$M | 0.0263 |
| $1 \times 10^{-7}$M | 0.0182 |
| $1 \times 10^{-6}$M | 0.0074 |
| $1 \times 10^{-5}$M | 0.0012 |

This example shows an improved change in rate as a function of the levels of $T_4$ measured.

Example 2 - Assay for Phenytoin

The following element is useful for assaying Phenytoin.

| | | Coverage (g/m²) Preferred | Ranges |
|---|---|---|---|
| Spreading Layer | Small beads (0.7μm) with covalently bound Phenytoin antibody receptors | 0.1 | 0.005–5 |
| | Large beads (30μm) | 130 | 25–200 |
| | Polyvinylpyrrolidone | 0.08 | 0.02–0.2 |
| | Triton X-100 | 0.05 | 0.02–0.2 |
| | Binder | 2 | 1–15 |
| Subbing Layer | Gelatin | 1 | 0.2–5 |
| | TEA, pH 7.5 | 0.2 | 0.05–1 |
| | BSA | 0.05 | 0.01–0.2 |
| | Zonyl FSN | 0.1 | 0.05–0.5 |
| | $MgCl_2$ | 0.01 | 0.005–0.05 |
| | $ZnCl_2$ | 0.02 | 0.005–0.05 |
| | Phenytoin-ALP conjugate | $7x10^{-5}$ or 0 | |
| Gelatin Layer | Hardened gelatin | 10 | 5–20 |
| | TEA, pH 7.5 | 0.8 | 0.01–1.0 |
| | Triton X-100 | 0.02 | 0.01–0.1 |

This example illustrates a competitive radial wash immunoassay for phenytoin using a three-layer format. A first series of aqueous solutions containing phenytoin standards in 0.15 M sodium chloride (NaCl) and 0.1% bovine serum albumin (BSA) was prepared. The concentration of phenytoin varied from 2 x $10^{-9}$M to 2 x $10^{-4}$M. These solutions were mixed (1:1) with buffer solution (0.02M MOPS, pH 7, 0.15M NaCl and 2% BSA). A second series of aqueous solutions were prepared as above and mixed (1:1) with buffer solution containing 10nM phenytoin-alkaline phosphatase conjugate.

The first series of phenytoin standards (10μL aliquots) was spotted onto the elements that contained the incorporated conjugate. The second series of phenytoin standards (10μL aliquots) was spotted onto elements that did not contain the incorporated conjugate. After 5 minutes incubation at 37°C, the wash solution (10μL) was added to the elements to wash the unbound conjugate away from the center of the spot. After one minute at 37°C, the change in reflection density ($\triangle D_R$) at 400mm was measured over 30 seconds in the center of the spot. Results are shown in Table 3.

Table 3

| Phenytoin Conc (Molar) | Rate ($\Delta D_R$/min) Conjugate Coated | Conjugate Spotted |
|---|---|---|
| $1 \times 10^{-9}$ | 0.0321 | 0.0361 |
| $1 \times 10^{-8}$ | 0.0314 | 0.0330 |
| $1 \times 10^{-7}$ | 0.0293 | 0.0315 |
| $1 \times 10^{-6}$ | 0.0209 | 0.0229 |
| $1 \times 10^{-5}$ | 0.0160 | 0.0076 |
| $1 \times 10^{-4}$ | 0.0044 | −0.0024 |

## Claims

1. An analytical element for immunoassays comprising a support and a spreading layer characterized in that the element contains,
   a) a first population of large polymeric beads having a diameter in the range of 10 to 200 μm to facilitate uniform spreading of liquids applied to the element; and
   b) a second population of small polymeric beads having (i) a diameter in the range of 0.1 to 5 μm; (ii) receptors covalently bound to the bead surface through surface reactive groups; and (iii) surfaces which are free of residual materials.

2. The element according to claim 1 comprising a reagent layer containing an indicator composition.

3. The element of claim 1 or 2 wherein the receptor is a protein.

4. The element of claim 3 wherein the receptor is an antibody.

5. The element of claim 4 wherein the antibody is a monoclonal antibody.

6. The element of claim 4 or 5 wherein the antibody is against a protein.

7. The element of claim 4 or 5 wherein the antibody is against a member selected from the group consisting of digoxin, thyroxine and phenytoin.

8. The element of claim 4 or 5 wherein the antibody is against a member selected from the group phenobarbital, theophylline, gentamicin, carbamazepine and tobramycin.

9. The element of claim 1 or 2 wherein the receptor is a member selected from the group consisting of digoxin, thyroxine and phenytoin.

10. The element of claim 1 or 2 wherein the receptor comprises a hapten selected from the group phenobarbital, theophylline, gentamicin, carbamazepine and tobramycin.

11. The element according to any one of the preceding claims wherein the spreading layer comprises both the first and second population of polymeric beads.

12. The element of claim 11 wherein the spreading layer comprises a) reagents for an indicator composition; b) a particulate structure containing the first population of polymeric beads, bonded to each other through surface areas of adjacent beads to form a coherent, three-dimensional latice that is essentially non-swellable in an aqueous liquid; and c) the second population of polymer beads.

13. The element according to any one of claims 1 to 10 wherein the second population of polymeric beads is located in a layer above or below the spreading layer.

14. A method for assaying an immunological reactive ligand in a liquid with an analytical element according to any one of the preceding claims comprising the steps of:
A. in the presence of a labeled ligand analog, contacting a finite area of the spreading layer with a sample of the liquid to form an immobilized ligand-receptor complex within the finite area and to effect horizontal separation of uncomplexed ligand from the immobilized complex during liquid spreading, and
B. after the completion of step A, determining the immobilized complex in the center of the finite area.

15. A method for assaying an immunologically reactive ligand in a liquid with an analytical element according to any one of claims 1 to 13 comprising the steps of:
A. in the presence of a labeled ligand analog, contacting a finite area of the spreading layer with a sample of the liquid to form an immobilized ligand-receptor complex within the finite area;
B. adding a wash solution to effect substantial horizontal separation of uncomplexed ligand from the immobilized complex; and
C. after the completion of step B, determining the immobilized complex in the center of the finite area.

## Patentansprüche

1. Analytisches Element für Immunoassays mit einem Träger und einer Ausbreitschicht, dadurch gekennzeichnet, daß das Element aufweist:
a) eine erste Population von großen polymeren Kügelchen mit einem Durchmesser im Bereich von 10 bis 200 μm zur Erleichterung einer gleichförmigen Ausbreitung von Flüssigkeit, die auf das Element aufgebracht wird; und
b) eine zweite Population von kleinen polymeren Teilchen mit (i) einem Durchmesser im Bereich von 0,1 bis 5 μm; (ii) Rezeptoren, die kovalent an die Oberfläche der Kügelchen durch reaktive Oberflächengruppen gebunden sind; und (iii) Oberflächen, die frei von restlichen Materialien sind.

2. Element nach Anspruch 1 mit einer Reagenzschicht mit einer Indikator-Zusammensetzung.

3. Element nach Anspruch 1 oder 2, in dem der Rezeptor ein Protein ist.

4. Element nach Anspruch 3, in dem der Rezeptor ein Antikörper ist.

5. Element nach Anspruch 4, in dem der Antikörper ein monoklonaler Antikörper ist.

6. Element nach Anspruch 4 oder 5, in dem der Antikörper gegen ein Protein gerichtet ist.

7. Element nach Anspruch 4 oder 5, in dem der Antikörper gegen ein Glied gerichtet ist, das ausgewählt ist aus der Gruppe bestehend aus Digoxin, Thyroxin und Phenytoin.

8. Element nach Anspruch 4 oder 5, in dem der Antikörper gegen ein Glied gerichtet ist, das ausgewählt ist aus der Gruppe bestehend aus Phenobarbital, Theophyllin, Gentamicin, Carbamazepin und Tobramycin.

9. Element nach Anspruch 1 oder 2, in dem der Rezeptor ein Glied ist, das ausgewählt ist aus der Gruppe bestehend aus Digoxin, Thyroxin und Phenytoin.

10. Element nach Anspruch 1 oder 2, in dem der Rezeptor ein Hapten umfaßt, das ausgewählt ist aus der Gruppe von Phenobarbital, Theophyllin, Gentamicin, Carbamazepin und Tobramycin.

11. Element nach einem der vorstehenden Ansprüche, in dem die Ausbreitschicht eine erste und zweite Population aus polymeren Kügelchen aufweist.

12. Element nach Anspruch 11, in dem die Ausbreitschicht umfaßt a) Reagenzien für eine Indikator-Zusammensetzung; b) eine teilchenförmige Struktur mit der ersten Population von polymeren Kügelchen, die aneinander durch Oberflächenbereiche von benachbarten Kügelchen gebunden sind, unter Ausbildung eines kohärenten, dreidimensionalen Gitters, das praktisch nicht-quellbar in einer wäßrigen Flüssigkeit ist; und c) die zweite Population von polymeren Kügelchen.

13. Element nach einem der Ansprüche 1 bis 10, in dem die zweite Population der polymeren Kügelchen sich in einer Schicht oberhalb oder unterhalb der Ausbreitschicht befindet.

14. Verfahren zur Bestimmung eines immunologisch reaktiven Liganden in einer Flüssigkeit mit einem analytischen Element nach einem der vorstehenden Ansprüche, mit den Stufen:
A. Kontaktieren, in Gegenwart eines markierten LigandenAnalogen, einer begrenzten Fläche der Ausbreitschicht mit einer Probe der Flüssigkeit unter Bildung eines immobilisierten Liganden-Rezeptor-Komplexes innerhalb des begrenzten Bereiches, und um eine horizontale Trennung der nicht komplex gebundenen Liganden von dem immobilisierten Komplex während der Flüssigkeits-Ausbreitung zu bewirken, und
B. Bestimmung des immobilisierten Komplexes in dem Zentrum der begrenzten Fläche nach Vervollständigung der Stufe A.

15. Verfahren zur Bestimmung eines immunologisch reaktiven Liganden in einer Flüssigkeit mit einem analytischen Element nach einem der Ansprüche 1 bis 13 mit den Stufen:
A. Kontaktieren, in Gegenwart eines markierten LigandenAnalogen, einer begrenzten Fläche der Ausbreitschicht mit einer Probe der Flüssigkeit unter Bildung eines immobilisierten Liganden-Rezeptor-Komplexes innerhalb der begrenzten Fläche;
B. Zugabe einer Waschlösung, um eine praktisch horizontale Trennung von nicht komplex gebundenem Liganden von dem immobilisierten Komplex zu bewirken; und
C. Bestimmung des immobilisierten Komplexes in dem Zentrum der begrenzten Fläche nach Vervollständigung der Stufe B.

## Revendications

1. Elément analytique pour immunoanalyses comprenant un support et une couche d'étalement, caractérisé en ce que l'élément contient :
a) une première population de billes polymériques de grande taille ayant un diamètre situé dans la plage de 10 à 200 μm pour facilier l'étalement uniforme des liquides appliqués à l'élément, et
b) une seconde population de billes polymériques de petite taille ayant (i) un diamètre situé dans la plage de 0,1 à 5 μm, (ii) des récepteurs liés de manière covalente à la surface des billes par l'intermédiaire de groupes réactifs superficiels et (iii) des surfaces qui sont exemptes de substances résiduelles.

2. Elément selon la revendication 1, comprenant une couche de réactifs contenant une composition indicatrice.

3. Elément selon la revendication 1 ou 2, dans lequel le récepteur est une protéine.

4. Elément selon la revendication 3, dans lequel le récepteur est un anticorps.

5. Elément selon la revendication 4, dans lequel l'anticorps est un anticorps monoclonal.

6. Elément selon la revendication 4 ou 5, dans lequel l'anticorps est dirigé contre une protéine.

7. Elément selon la revendication 4 ou 5, dans lequel l'anticorps est dirigé contre un élément choisi dans le groupe formé par la digoxine, la thyroxine et la phénytoïne.

8. Elément selon la revendication 4 ou 5, dans lequel l'anticorps est dirigé contre un élément choisi dans le groupe formé par le phénobarbital, la théophylline, la gentamicine, la carbamazépine et la tobramycine.

9. Elément selon la revendication 1 ou 2, dans lequel le récepteur est un élément choisi dans le groupe formé par la digoxine, la thyroxine et la phénytoïne.

10. Elément selon la revendication 1 ou 2, dans lequel le récepteur comprend un haptène choisi dans le groupe formé par le phénobarbital, la théophylline, la gentamicine, la carbamazépine et la tobramycine.

11. Elément selon l'une quelconque des revendications précédentes, dans lequel la couche d'établement comprend les première et seconde populations de billes polymériques.

12. Elément selon la revendication 11, dans lequel la couche d'étalement comprend a) des réactifs pour une composition indicatrice, b) une structure particulaire contenant la première population de billes polymériques liées les unes aux autres par des zones de surface des billes adjacentes pour former un réseau

tridimensionnel cohérent qui est sensiblement non gonflable dans un liquide aqueux, et c) la seconde population de billes polymériques.

**13.** Elément selon l'une quelconque des revendications 1 à 10, dans lequel la seconde population de billes polymériques est située dans une couche supérieure ou inférieure à la couche d'étalement.

**14.** Procédé pour déterminer un ligand immunologiquent réactif dans un liquide avec un élément analytique selon l'une quelconque des revendications précédentes, comprenant les étapes suivantes :
A. en présence d'un analogue de ligand marqué, mise en contact d'une zone finie de la couche d'étalement avec un échantillon du liquide pour former un complexe ligand-récepteur immobilisé dans la zone finie et pour réaliser une séparation horizontale du ligand non complexé et du complexe immobilisé pendant l'étalement du liquide, et
B. après l'achèvement de l'étape A, détermination du complexe immobilisé au centre de la zone finie.

**15.** Procédé pour déterminer un ligand immunologiquement réactif dans un liquide avec un élément analytique selon l'une quelconque des revendications 1 à 13, comprenant les étapes suivantes :
A. en présence d'un analogue de ligand marqué, mise en contact d'une zone finie de la couche d'étalement avec un échantillon du liquide pour former un complexe ligand-récepteur immobilisé dans la zone finie,
B. addition d'une solution de lavage pour réaliser une séparation horizontale sensible du ligand non complexé et du complexe immobilisé, et
C. après l'achèvement de l'étape B, détermination du complexe immobilisé au centre de la zone finie.